Europäisches Patentamt

European Patent Office

Office européen des brevets

(19)

(11) **EP 0 999 182 B1**

(12) **FASCICULE DE BREVET EUROPEEN**

(45) Date de publication et mention
de la délivrance du brevet:
**14.05.2003 Bulletin 2003/20**

(51) Int Cl.⁷: **C01B 39/48**, B01D 15/00,
B01J 29/70, C10G 45/64,
C10G 35/095, C10G 49/08

(21) Numéro de dépôt: **99402632.6**

(22) Date de dépôt: **21.10.1999**

(54) **Procédé de préparation d'une zéolithe de type structural EUO à l'aide de germes de matériaux zéolitiques et son utilisation comme catalyseur d'isomérisation des aromatiques à huit atomes de carbone**

Verfahren zur Herstellung eines Zeoliths des EUO-Typs mittels zeolithischen Keimen und dessen Verwendung als Isomerisierungskatalysator von Aromaten mit acht Kohlenstoffatomen

Process for the preparation of EUO-type zeolite using zeolitic seeds and use thereof as catalyst for isomerizing eight carbon-aromatics

(84) Etats contractants désignés:
**DE DK GB NL**

(30) Priorité: **02.11.1998 FR 9813839**
**23.12.1998 FR 9816412**

(43) Date de publication de la demande:
**10.05.2000 Bulletin 2000/19**

(73) Titulaire: **INSTITUT FRANCAIS DU PETROLE**
**92852 Rueil-Malmaison Cedex (FR)**

(72) Inventeurs:
  • **Rouleau, Loic**
    **69600 Oullins (FR)**
  • **Lacombe, Sylvie**
    **92500 Rueil-Malmaison (FR)**
  • **Merlen, Elisabeth**
    **92500 Rueil-Malmaison (FR)**
  • **Alario, Fabio**
    **92200 Neuilly sur Seine (FR)**
  • **Kolenda, Frédéric**
    **69630 Chaponost (FR)**

(56) Documents cités:
  **EP-A- 0 042 226          EP-A- 0 051 318**
  **US-A- 4 275 047**

  • **CASCI J L ET AL: "SYNTHESIS AND**
    **CHARACTERISATION OF ZEOLITE EU-1"**
    **PROCEEDINGS OF THE SIXTH INTERNATIONAL**
    **ZEOLITE CONFERENCE.;RENO, NV, USA,1984,**
    **pages 894-904, XP002108428 1984 Engl,**
    **Butterworths & Co, Guildford, Engl**

## Description

La présente invention concerne un nouveau procédé de préparation d'un matériau zéolithique de type structural EUO. Les zéolithes de type structural EUO comprennent la zéolithe EU-1, la zéolithe TPZ-3 et la zéolithe ZSM-50 et ont généralement la formule suivante sous forme anhydre : 0 à 20 $R_2O$ : 0-10 $T_2O_3$ : 0-100 $XO_2$ : où R représente un cation monovalent ou 1/n d'un cation de valence n, X représente le silicium et/ou le germanium, T représente au moins un élément choisi parmi l'aluminium, le fer, le gallium, le bore, le titane, le vanadium, le zirconium, le molybdène, l'arsenic, l'antimoine, le chrome et le manganèse.

Les zéolithes de type structural EUO sont généralement synthétisées par mélange en milieu aqueux d'une source de silice et/ou de germanium et d'une source d'au moins un élément choisi parmi l'aluminium, le fer, le gallium, le bore, le titane, le vanadium, le zirconium, le molybdène, l'arsenic, l'antimoine, le chrome et le manganèse en présence d'au moins un composé organique azoté jouant le rôle de structurant, ou des produits de dégradation correspondant ou des précurseurs. Le mélange est généralement maintenu à une certaine température jusqu'à la cristallisation de la zéolithe.

La présente invention concerne également un procédé de préparation d'un catalyseur à base de zéolithe de structure EUO, ladite zéolithe étant obtenue selon un nouveau mode de synthèse. L'invention concerne également un procédé d'isomérisation des composés aromatiques à 8 atomes de carbone encore appelés « coupes C8 aromatiques » en présence dudit catalyseur à base de zéolithe de type structural EUO.

L'isomérisation en xylènes de l'éthylbenzène nécessite la présence d'un métal du groupe VIII. Les formulations optimisées à base de mordénite et d'un métal du groupe VIII conduisent à des catalyseurs avec lesquels les réactions parasites restent non négligeables. Par exemple, on peut citer l'ouverture de cycles naphténiques suivie ou non de craquage ou encore les réactions de dismutation et de transalkylation des aromatiques en C8 qui conduisent à la formation d'aromatiques non recherchés. Il est donc particulièrement intéressant de trouver de nouveaux catalyseurs plus sélectifs.

### Art antérieur

La zéolithe EU-1 de type structural EUO, déjà décrite dans l'art antérieur, présente un réseau microporeux monodimensionnel, dont le diamètre des pores est de 4,1 x 5,7 Å (1 Å = 1 Angström = $1.10^{-10}$ m) (« Atlas of Zeolites Structure types », W.M. Meier et D.H. Oison, 4ème Edition, 1996). D'autre part, N.A. Briscoe *et al* ont enseigné dans un article de la revue Zeolites (1988, 8, 74) que ces canaux monodimensionnels possèdent des poches latérales de profondeur 8,1 Å et de diamètre 6,8 x 5,8 Å. Le mode de synthèse de la zéolithe EU-1 et ses caractéristiques physico-chimiques ont été décrits dans le brevet EP 42 226. Le mode de synthèse comprend le mélange d'un oxyde de silicium et/ou de germanium et d'un oxyde d'au moins un élément choisi parmi l'aluminium, le fer, le gallium et le bore, en présence d'un structurant comprenant au moins un dérivé alkylé d'une polyméthylène α-ω diammonium, un produit de dégradation dudit dérivé ou des précurseurs dudit dérivé.

La synthèse de la zéolithe EU-1 à partir de germes de silicalite ou de EU-1 est décrite dans la littérature (Casci, J.L. ; Whittam, T.V. ; Lowe, B.M., Proc. Int. Zeolite Conf., 6 th (1984), Meeting Date 1983, 984-904. Editors Olson, David, Bisio, Attilio, Publisher: Butterworth, Guildfold, UK).

Le brevet US-4 640 829 concerne la zéolithe ZSM-50, qui d'après "l'Atlas of Zeolites Structure types", W.M. Meier et D.H. Olson, 4ème Edition, 1996, présente le type structural EUO. Ledit brevet présente un mode de synthèse de la ZSM-50 comprenant le mélange d'une source d'ions de métal alcalin, d'ions dibenzyldiméthylammonium ou de précurseurs de ceux-ci, d'oxyde de silicium, d'oxyde d'aluminium et d'eau.

La demande de brevet EP 51 318 traite de la zéolithe TPZ-3, qui présente d'après "l'Atlas of Zeolites Structure types", W.M. Meier et D.H. Olson, 4ème Edition, 1996, le même type structural EUO que la zéolithe EU-1. La préparation de la zéolithe comprend le mélange d'un composé de métal alcalin soluble, un composé de 1,6-N,N,N,N',N',N'-hexaméthylhexaméthylènediammonium, un composé capable de donner de la silice et un composé capable de donner de l'alumine, à une température supérieure à 80°C.

### Résumé de l'invention

La présente invention concerne un procédé de préparation d'une zéolithe de type structural EUO comprenant au moins un élément X choisi parmi le silicium et le germanium et au moins un élément T choisi parmi le fer, l'aluminium, le gallium, le bore, le titane, le vanadium, le zirconium, le molybdène, l'arsenic, l'antimoine, le chrome et le manganèse, caractérisé en ce qu'on utilise des germes d'au moins un matériau zéolithique comprenant au moins un élément X' choisi parmi le silicium et le germanium et au moins un élément T' choisi parmi le fer, l'aluminium, le gallium, le bore, le titane, le vanadium, le zirconium, le molybdène, l'arsenic, l'antimoine, le chrome et le manganèse, lesdits germes étant différents de la zéolithe de type structural EUO que l'on prépare. Les germes de zéolithe mis en oeuvre ont un rapport X'/T' inférieur à 200. La présente invention concerne également l'utilisation de la zéolithe EUO comme catalyseur, ledit catalyseur comprenant en outre au moins un métal du groupe VIII de la classification périodiques des
éléments et pouvant être utilisé dans des réactions d'isomérisation des composés aromatiques ayant 8 atomes de
carbone par molécule.

Intérêt de l'invention

Le procédé selon l'invention permet de réduire le temps de cristallisation de la zéolithe EUO après formation
du mélange et permet d'atteindre un rendement maximal en produit pur, ce qui procure un gain de coût. La composition
du milieu réactionnel peut être plus large, ce qui procure un gain de flexibilité.
Ainsi, la demanderesse a découvert que la synthèse d'une zéolithe de type structural EUO caractérisée par
l'utilisation de germes d'au moins un matériau zéolithique différent du matériau que l'on cherche à synthétiser permettait
notamment d'obtenir les avantages cités ci-dessus, c'est-à-dire un gain de temps, de rendement en produit plus pur
et une flexibilité de composition du mélange réactionnel.

Description de l'invention

La présente invention concerne un procédé de préparation d'un matériau zéolithique de type structural EUO
comprenant au moins un élément X choisi parmi le silicium et le germanium et au moins un élément T choisi parmi le
fer, l'aluminium, le gallium, le bore, le titane, le vanadium, le zirconium, le molybdène, l'arsenic, l'antimoine, le chrome
et le manganèse, caractérisé en ce qu'on utilise des germes d'au moins un matériau zéolithique comprenant au moins
un élément X' choisi parmi le silicium et le germanium et au moins un élément T' choisi parmi le fer, l'aluminium, le
gallium, le bore, le titane, le vanadium, le zirconium, le molybdène, l'arsenic, l'antimoine, le chrome et le manganèse,
lesdits germes étant différents de la zéolithe de type structural EUO que l'on prépare. Les germes de zéolithe mis en
oeuvre ont un rapport X'/T' inférieur à 200.
La différence entre la zéolithe de type structural EUO que l'on cherche à synthétiser et le matériau zéolithique
introduit en tant que germes se situe soit dans la différence de type structural, soit dans la différence de composition
chimique de la charpente cristalline, soit dans la différence de type structural et la différence de composition chimique
de la charpente cristalline.
Le procédé de préparation selon l'invention comprend le mélange en milieu aqueux d'au moins une source
d'au moins un élément X choisi parmi le silicium et le germanium, d'au moins une source d'au moins un élément T
choisi parmi l'aluminium le fer, le gallium, le bore, le titane, le vanadium, le zirconium, le molybdène, l'arsenic, l'antimoine, le chrome et le manganèse, d'au moins un composé organique azoté Q choisi parmi les dérivés alkylés de
polyméthylène $\alpha$-$\omega$ diammonium, les sels de dibenzyldiméthylammonium, un produit de dégradation d'amine correspondant audit composé organique Q, des précurseurs correspondants audit composé organique Q, et des germes de
matériau zéolithique S. L'invention est caractérisée en ce qu'on utilise en tant que germes au moins un matériau
zéolithique différent de la zéolithe EUO que l'on cherche à préparer. La réaction du mélange est maintenue jusqu'à
cristallisation de la zéolithe.
Le dérivé alkylé de polyméthylène $\alpha$-$\omega$ diammonium, jouant le rôle de structurant organique, utilisé plus particulièrement pour la synthèse de la zéolithe EU-1 ou de la zéolithe TPZ-3, est défini par la formule : $R_1R_2R_3 N^+ (CH_2)_n$
$N^+ R_4R_5R_6$, n étant compris entre 3 et 12 et $R_1$ à $R_6$, identiques ou différents, pouvant représenter des radicaux alkyles
ou hydroxyalkyles ayant de 1 à 8 atomes de carbone, jusqu'à cinq radicaux $R_1$ à $R_6$ pouvant être de l'hydrogène.
Ainsi, on pourra utiliser des germes d'au moins un solide de type structural LTA telle la zéolithe A, de type
structural LTL, de type structural FAU telles les zéolithes X et Y, de type structural MOR, de type structural MAZ, de
type structural OFF, de type structural FER, de type structural ERI, de type structural BEA, de type structural MFI telles
la ZSM-5 et la silicalite, de type structural MTW, de type structural MTT, de type structural LEV, de type structural TON,
de type structural NES telle la zéolithe NU-87, ou une zéolithe NU-85, NU-86, NU-88, IM-5 ou une zéolithe de type
structural EUO de composition chimique de la charpente cristalline différente de la composition chimique de la charpente cristalline de la zéolithe EUO que l'on prépare, en particulier avec différents rapports X'/T', ceux-ci étant inférieur
à 200. De préférence on utilise comme germes pour la synthèse d'une zéolithe de type structural EUO des germes de
zéolithes de type structural LTA, FAU, MOR, MFI, EUO de rapport X'/T' inférieur à 200.
Les matériaux zéolithiques jouant le rôle de germes peuvent être introduits à n'importe quel moment de la
préparation de la zéolithe que l'on cherche à synthétiser. Les germes peuvent être introduits en même temps que les
sources des éléments X et T, que le structurant organique Q, ou les germes peuvent être introduits en premier dans
le mélange aqueux ou encore les germes peuvent être introduits après l'introduction des sources des éléments X et
T et du structurant. De préférence, les germes sont introduits après homogénéisation au moins en partie du mélange
aqueux contenant les sources des éléments X et T et le structurant.
Les matériaux zéolithiques jouant le rôle de germes peuvent être introduits lors de la synthèse de la zéolithe que l'on cherche à synthétiser sous plusieurs formes. Ainsi les germes peuvent être introduits, après avoir subi au moins l'une des étapes choisie parmi les étapes suivantes : lavage, séchage, calcination et échange ionique. Les germes peuvent également être introduits sous la forme brute de synthèse.

La taille des particules de germes peut avoir une influence sur le processus de synthèse et doit présenter de préférence le calibre souhaité. On entend par particule de germes de zéolithe, soit un cristal de zéolithe soit un agrégat de cristaux de zéolithe. Ainsi, au moins la majeure partie des particules de germes introduites lors de la préparation de matériau zéolithique ont une taille comprise entre 0,001 et 500 µm, de préférence entre 0,005 et 250 µm.

Dans une mise en oeuvre particulière, indépendante ou non de la mise en oeuvre précédente, il peut être avantageux d'ajouter au milieu réactionnel au moins un sel de métal alcalin ou d'ammonium P. On peut citer par exemple des radicaux acides forts tels que du bromure, du chlorure, de l'iodure, du sulfate, du phosphate ou du nitrate, ou des radicaux acides faibles tels que les radicaux acides organiques, par exemple du citrate ou de l'acétate. Ce sel peut accélérer la cristallisation des zéolithes EUO à partir du mélange réactionnel.

Dans le procédé selon l'invention, le mélange réactionnel a la composition suivante, exprimée sous la forme d'oxyde :

| | |
|---|---|
| $XO_2/T_2O_3$ (mol/mol) | au moins 10 |
| $OH^-/XO_2$ (mol/mol) | 0,002 à 2,0 |
| $Q/XO_2$ (mol/mol) | 0,002 à 2 |
| $Q/(M^+ + Q)$ (mol/mol) | 0,1 à 1,0 |
| $H_2O/XO_2$ (mol/mol) | 1 à 500 |
| $P/XO_2$ (mol/mol) | 0 à 5 |
| $S/XO_2$ (g/g) | 0,0001 à 0,1 |

de manière préférée, le mélange réactionnel a la composition suivante, exprimée sous la forme d'oxydes :

| | |
|---|---|
| $XO_2/T_2O_3$ (mol/mol) | au moins 12 |
| $OH^-/XO_2$ (mol/mol) | 0,005 à 1,5 |
| $Q/XO_2$ (mol/mol) | 0,005 à 1,5 |
| $Q/(M^+ + Q)$ (mol/mol) | 0,1 à 1,0 |
| $H_2O/XO_2$ (mol/mol) | 3 à 250 |
| $P/XO_2$ (mol/mol) | 0 à 1 |
| $S/XO_2$ (g/g) | 0,0005 à 0,07 |

et, de manière encore plus préférée, le mélange réactionnel a la composition suivante, exprimée sous la forme d'oxydes :

| | |
|---|---|
| $XO_2/T_2O_3$ (mol/mol) | au moins 15 |
| $OH^-/XO_2$ (mol/mol) | 0,01 à 1 |
| $Q/XO_2$ (mol/mol) | 0,01 à 1 |
| $Q/(M^+ + Q)$ (mol/mol) | 0,1 à 1,0 |
| $H_2O/XO_2$ (mol/mol) | 5 à 100 |
| $P/XO_2$ (mol/mol) | 0 à 0,25 |
| $S/XO_2$ (g/g) | 0,001 à 0,04 |

Où

X est le silicium et/ou le germanium,

T est au moins un élément choisi parmi l'aluminium, le fer, le gallium, le bore, le titane, le vanadium, le zirconium, le molybdène, l'arsenic, l'antimoine, le chrome et le manganèse,

$M^+$ représente un métal alcalin ou l'ion ammonium,

Q représente le structurant organique, ou les produits de décomposition correspondants audit dérivé ou les précurseurs dudit dérivé,

S représente les germes de zéolithe présents sous la forme brute, séchée, calcinée ou échangée, avec un rapport X'/T' inférieur à 200,

P représente le sel de métal alcalin ou d'ammonium.

M et/ou Q peuvent être présents sous forme d'hydroxydes ou de sels d'acides inorganiques ou organiques à la condition que le critère OH⁻ / XO$_2$ soit satisfait.

La quantité de germes introduite par rapport à la quantité d'oxyde XO$_2$ est comprise entre 0,001 et 10 %, et de préférence entre 0,05 et 7 %, et de manière encore plus préférée entre 0,1 et 4 %.

La synthèse de la zéolithe EUO selon le procédé de la présente invention est effectuée à l'aide d'un composé organique Q jouant le rôle de structurant.

Dans le cas de la synthèse de la zéolithe EU-1, les dérivés alkylés de α-ω polyméthylènediammonium Q préférés de départ sont, entre autres, les dérivés alkylés d'hexaméthylène α-ω diammonium et spécialement les dérivés méthylés d'hexaméthylène α-ω diammonium et encore plus préférentiellement les sels de 1,6 N,N,N,N',N',N'-hexaméthylhexaméthylène α-ω diammonium, par exemple l'halogénure, l'hydroxyde, le sulfate, le silicate, l'aluminate.

Les dérivés alkylés de polyméthylène α-ω diammonium peuvent être obtenus à partir de précurseurs. Les précurseurs appropriés des dérivés alkylés de polyméthylène α-ω diammonium de départ sont notamment les diamines apparentées conjointement avec des alcools, des halogénures d'alkyles, des alcanediols ou des dihalogénures d'alcane apparentés conjointement avec des alkylamines. Ils peuvent être mélangés tels quels avec les autres réactifs ou ils peuvent être préchauffés ensemble dans le récipient de réaction de préférence en solution avant l'addition des autres réactifs nécessaires pour la synthèse de la zéolithe EU-1.

Dans le cas de la préparation de la zéolithe ZSM-50, le structurant organique Q peut être un sel de dibenzyldiméthylammonium tel l'halogénure, l'hydroxyde, le sulfate, le silicate ou l'aluminate.

Les sels de dibenzyldiméthylammonium peuvent être obtenus à partir de précurseurs. Les précurseurs appropriés sont la benzyldiméthylamine et l'halogénure de benzyle ou l'alcool benzylique. Ils peuvent être utilisés tels quels in situ ou peuvent être préchauffés ensemble dans le récipient de réaction de préférence en solution avant l'addition des autres réactifs nécessaires pour la synthèse de la zéolithe ZSM-50.

Le métal alcalin (M⁺) préféré est le sodium. L'élément préféré X est le silicium. L'élément T préféré est l'aluminium.

La source de silicium peut être l'une quelconque de celles dont l'utilisation est normalement envisagée pour la synthèse des zéolithes, par exemple la silice solide en poudre, l'acide silicique, la silice colloïdale ou la silice en solution. Parmi les silices en poudre utilisables, il convient de citer les silices précipitées, spécialement celles obtenues par précipitation à partir d'une solution d'un silicate de métal alcalin, comme les « Zeosil » ou les « Tixosil », produites par Rhône-Poulenc, les silices pyrogénées telles que les « Aerosil » produites par Degussa et les « Cabosil » produites par Cabot et les gels de silice. Des silices colloïdales de diverses granulométries peuvent être utilisées, comme celles vendues sous les marques déposées « LUDOX » de Dupont, et « SYTON » de Monsantos.

Les silices dissoutes utilisables sont notamment les verres solubles ou silicates commercialisés contenant : 0,5 à 6,0 et spécialement 2,0 à 4,0 moles de SiO$_2$ par mole d'oxyde de métal alcalin et les silicates obtenues par dissolution de silice dans un hydroxyde de métal alcalin, un hydroxyde d'ammonium quaternaire ou un mélange de ceux-ci.

La source d'aluminium est le plus avantageusement l'aluminate de sodium, mais peut aussi être l'aluminium, un sel d'aluminium, par exemple le chlorure, le nitrate ou le sulfate, un alcoolate d'aluminium ou l'alumine elle-même qui devrait de préférence se trouver sous une forme hydratée ou hydratable comme l'alumine colloïdale, la pseudoboehmite, la boehmite, l'alumine gamma, ou les trihydrates.

On peut utiliser des mélanges des sources citées ci-dessus. Des sources combinées de silicium et d'aluminium peuvent aussi être mises en oeuvre telles que les silice-alumines amorphes ou certaines argiles.

Le mélange de réaction est habituellement mis à réagir sous la pression autogène, éventuellement avec apport d'un gaz, par exemple d'azote, à une température comprise entre 85 et 250°C jusqu'à ce qu'il se forme des cristaux de la zéolithe, ce qui peut durer de 1 minute à plusieurs mois suivant la composition des réactifs, le mode de chauffage et de mélange, la température de travail et l'agitation. L'agitation est facultative, mais préférable, parce qu'elle abrège la durée de réaction.

Au terme de la réaction, la phase solide est collectée sur un filtre et lavée et est alors prête pour les opérations suivantes comme le séchage, la calcination et l'échange d'ions.

Ainsi, afin d'obtenir la forme hydrogène de la zéolithe de structure EUO, on peut effectuer un échange d'ions avec un acide, spécialement un acide minéral fort comme l'acide chlorhydrique, sulfurique ou nitrique, ou avec un composé tel que le chlorure, le sulfate ou le nitrate d'ammonium. L'échange d'ions peut être effectué par dilution en une ou plusieurs fois avec la solution d'échange d'ions. La zéolithe peut être calcinée avant ou après l'échange d'ions, ou entre deux étapes d'échange d'ions, de préférence avant l'échange d'ions afin d'éliminer toute substance organique absorbée, dans la mesure où l'échange d'ions s'en trouve facilité.

En règle générale, le ou les cations de la zéolithe de type structural EUO, peuvent être remplacés par un ou des cations quelconques de métaux et en particulier ceux des groupes IA, IB, IIA, IIB, IIIA, IIIB (y compris les terres rares), VIII (y compris les métaux nobles) de même que par le plomb, l'étain et le bismuth (Le tableau périodique est tel que dans "Handbook of Physic and Chemistry", 76ème édition). L'échange est exécuté au moyen de sels hydrosolubles quelconques contenant le cation approprié.

**EP 0 999 182 B1**

La présente invention concerne également l'utilisation de la zéolithe telle que préparée selon le procédé de la présente invention comme adsorbant pour le contrôle de la pollution, comme tamis moléculaire pour la séparation et comme solide acide pour la catalyse dans les domaines du raffinage et de la pétrochimie.

Par exemple, lorsqu'elle est utilisée comme catalyseur, la zéolithe de structure EUO peut être associée à une matrice inorganique qui peut être inerte ou catalytiquement active et à une phase métallique. La matrice inorganique peut être présente simplement comme liant pour maintenir ensemble les petites particules de la zéolithe sous les différentes formes connues des catalyseurs (extrudés, billes, poudres), ou bien peut être ajoutée comme diluant pour imposer le degré de conversion dans un procédé qui progresserait sinon à une allure trop rapide conduisant à un encrassement du catalyseur en conséquence d'une formation exagérée de coke. Des matrices inorganiques typiques sont notamment des matières de support pour les catalyseurs comme la silice, les différentes formes d'alumine, et les argiles kaoliniques, les bentonites, les montmorillonites, la sépiolite, l'attapulgite, la terre à foulon, les matières poreuses synthétiques comme $SiO_2$-$Al_2O_3$, $SiO_2$-$ZrO_2$, $SiO_2$-$ThO_2$, $SiO_2$-BeO, $SiO_2$-$TiO_2$ ou toute combinaison de ces composés.

La zéolithe de type structural EUO peut aussi être associée à au moins une autre zéolithe et jouer le rôle de phase active principale ou d'additif.

La matrice inorganique peut être un mélange de différents composés, en particulier d'une phase inerte et d'une phase inorganique.

La phase métallique est introduite sur la zéolithe seule, la matrice inorganique seule ou l'ensemble matrice inorganique-zéolithe par échange d'ions ou imprégnation avec des cations ou oxydes choisis parmi les suivants, Cu, Ag, Ga, Mg, Ca, Sr, Zn, Cd, B, Al, Sn, Pb, V, P, Sb, Cr, Mo, W, Mn, Re, Fe, Co, Ni, Pt, Pd, Ru, Rh, Os, Ir et tout autre élément de la classification périodique des éléments.

Les compositions catalytiques comportant la zéolithe de type structural EUO peuvent trouver leur application dans les réactions d'isomérisation, de transalkylation et de dismutation, d'alkylation et de désalkylation, d'hydratation et de déshydratation, d'oligomérisation et de polymérisation, de cyclisation, d'aromatisation, de craquage et d'hydrocraquage, de reformage, d'hydrogénation et de déshydrogénation, d'oxydation, d'halogénation, de synthèses d'amines, d'hydrodésulfuration et d'hydrodénitrification, d'élimination catalytique des oxydes d'azote, la formation d'éther et la conversion d'hydrocarbures et la synthèse de composés organiques en général, lesdites réactions comprenant des hydrocarbures aliphatiques saturés et insaturés, des hydrocarbures aromatiques, des composés organiques oxygénés et des composés organiques contenant de l'azote et/ou du soufre, ainsi que des composés organiques contenant d'autres groupes fonctionnels.

De manière particulière, la présente invention concerne également l'utilisation de la zéolithe EUO dans un catalyseur d'isomérisation des composés aromatiques à 8 atomes de carbone, ledit catalyseur comprenant en outre au moins un élément du groupe VIII de la classification périodique et au moins un liant

Le catalyseur selon la présente invention, mis sous forme de billes ou d'extrudés, contient donc :

- au moins une zéolithe de type structural EUO, par exemple la zéolithe EU-1, synthétisée en présence de germes selon la méthode décrite précédemment,
- au moins un métal du groupe VIII de la classification périodique des éléments, choisi de préférence dans le groupe constitué par le palladium et le platine et de manière encore plus préférée le platine,
- au moins un liant, de préférence l'alumine,
- éventuellement au moins un métal appartenant au groupe formé par les éléments des groupes IB, IIB, IIIA, IVA, VIB et VIIB de la classification périodique des éléments, de préférence l'étain ou l'indium,
- éventuellement du soufre,

ledit catalyseur étant caractérisé en ce qu'il est préparé selon un nouveau mode de synthèse de la zéolithe de type EUO comprise dans le catalyseur qui permet de réduire son temps de synthèse avec un rendement maximal en produit pur ce qui réduit le coût de fabrication.

De manière plus précise, le catalyseur préparé selon le procédé de la présente invention, comprend généralement par rapport au poids de catalyseur :

- de 1 à 90 %, de préférence de 3 à 60 % et de manière encore plus préférée de 4 à 40 % en poids, d'au moins une zéolithe de structure EUO, obtenue selon le nouveau mode de synthèse, comprenant au moins un élément X choisi parmi le germanium et le silicium et au moins un élément T choisi dans le groupe formé par l'aluminium, le fer, le gallium, le bore, le titane, le vanadium, le zirconium, le molybdène, l'arsenic, l'antimoine, le chrome et le manganèse, de préférence l'aluminium et le bore, dont le rapport atomique X/T est supérieur ou égal à 5. La dite zéolithe est au moins en partie sous forme acide, c'est-à-dire sous forme hydrogène ($H^+$), la teneur en sodium étant telle que le rapport atomique Na/T est inférieur à 0,5, de préférence inférieur à 0,1, de manière encore plus préférée inférieur à 0,02,

- de 0,01 à 2 % et de préférence de 0,05 à 1,0 % en poids, d'au moins un métal du groupe VIII de la classification périodique des éléments, de préférence choisi dans le groupe formé par le platine et le palladium et de manière encore plus préférée le platine,
- éventuellement de 0,01 à 2 et de préférence entre 0,05 et 1,0 % en poids, d'au moins un métal du groupe formé par les groupes IB, IIB, IIIA, IVA, VIB et VIIB de la classification périodique des éléments, de préférence choisi dans le groupe formé par l'étain et l'indium,
- éventuellement du soufre dont la teneur est telle que le rapport du nombre d'atomes de soufre sur le nombre d'atomes de métal du groupe VIII déposés est compris entre 0,5 et 2 bornes incluses,
- le complément à 100 % en poids d'au moins un liant, de préférence de l'alumine.

Toute zéolithe de structure EUO connue de l'homme du métier et obtenue selon le mode de synthèse décrit dans le présent brevet convient pour le catalyseur utilisé dans la présente invention. Ainsi par exemple, la zéolithe utilisée comme base pour préparer ledit catalyseur peut être la zéolithe EU-1 brute de synthèse ayant les spécificités requises concernant le rapport X/T. On pourra procéder généralement à une calcination, puis à au moins un échange ionique dans au moins une solution de $NH_4NO_3$ de manière à obtenir une zéolithe dont la teneur en sodium résiduel est plus ou moins importante.

Le liant (ou matrice) compris dans le catalyseur préparé selon le procédé de la présente invention consiste généralement en au moins un élément choisi dans le groupe formé par les argiles, la magnésie, les alumines, les silices, l'oxyde de titane, l'oxyde de bore, la zircone, les phosphates d'aluminium, les phosphates de titane, les phosphates de zirconium et les silices alumines. On peut aussi utiliser du charbon. De préférence le liant est une alumine.

La zéolithe de type structural EUO, par exemple la zéolithe EU-1, comprise dans le catalyseur selon l'invention, est au moins en partie, de préférence pratiquement totalement, sous forme acide, c'est-à-dire sous forme hydrogène ($H^+$), la teneur en sodium étant de préférence telle que le rapport atomique Na/T est inférieur à 0,5, de préférence inférieur à 0,1, de manière encore plus préférée inférieur à 0,02.

Les métaux peuvent être introduits soit tous de la même façon soit par des techniques différentes, à tout moment de la préparation, avant ou après mise en forme et dans n'importe quel ordre. De plus, des traitements intermédiaires tels que par exemple une calcination et/ou une réduction peuvent être appliqués entre les dépôts des différents métaux.

La préparation de la matrice, l'introduction des métaux et la mise en forme du catalyseur pourront être effectuées par toute méthode connue de l'homme du métier. Ainsi au moins un élément du groupe VIII est introduit dans la zéolithe ou sur le liant, de préférence sur le liant avant ou après mise en forme.

Une méthode préférée consiste à réaliser un mélange de la matrice et de la zéolithe suivie d'une mise en forme. La mise en forme est généralement suivie d'une calcination, généralement à une température comprise entre 250°C et 600°C. Au moins un élément du groupe VIII de la classification périodique des éléments est introduit après cette calcination, de préférence par dépôt sélectif sur le liant. Lesdits éléments sont déposés pratiquement à plus de 90 %, totalement sur le liant de la manière connue de l'homme du métier par contrôle des paramètres utilisés lors dudit dépôt, comme par exemple la nature du précurseur utilisé pour effectuer ledit dépôt. Eventuellement, au moins un élément appartenant au groupe formé par les éléments des groupes IB, IIB, IIIA, IVA, VIB et VIIB est ajouté. On peut ajouter les éléments du groupe VIII et des groupes IB, IIB, IIIA, IVA, VIB et VIIB soit séparément à n'importe quelle étape de la préparation dudit catalyseur, soit simultanément dans au moins une étape unitaire. Lorsqu'un élément au moins des groupes IB, IIB, IIIA, IVA, VIB et VIIB est ajouté séparément, il est préférable qu'il soit ajouté préalablement à l'élément du groupe VIII.

Au moins un élément du groupe VIII est déposé de manière préférée sur le mélange zéolithe-liant préalablement mis en forme par tout procédé connu de l'homme du métier. Un tel dépôt est par exemple effectué par la technique d'imprégnation à sec, d'imprégnation par excès ou d'échange ionique. Tous les précurseurs conviennent pour le dépôt de ces éléments. Par exemple, de préférence, on mettra en oeuvre un échange anionique avec de l'acide hexachloroplatinique et/ou de l'acide hexachloropalladique en présence d'un agent compétiteur, par exemple l'acide chlorhydrique. Avec de tels précurseurs, le métal est pratiquement à plus de 90 % déposé totalement sur le liant et il présente une bonne dispersion et une bonne répartition macroscopique à travers le grain de catalyseur ce qui constitue une méthode préférée de préparation.

Eventuellement au moins un autre métal choisi dans le groupe formé par les éléments des groupes IB, IIB, IIIA, IVA, VIB et VIIB de la classification périodique des éléments est également introduit. Toutes les techniques de dépôt connues de l'homme du métier et tous les précurseurs conviennent pour l'introduction d'au moins un métal additionnel.

Une des méthodes préférées de préparation du catalyseur, utilisée dans la présente invention consiste à malaxer la zéolithe dans un gel humide de matrice (obtenu généralement par mélange d'au moins un acide et d'une poudre de matrice), par exemple l'alumine, pendant une durée nécessaire à l'obtention d'une bonne homogénéité de la pâte ainsi obtenue, soit par exemple pendant une dizaine de minutes, puis à passer la pâte à travers une filière pour former des extrudés, par exemple de diamètre compris entre 0,4 et 4 mm, bornes incluses. Puis après séchage, par exemple pendant quelques heures à environ 120°C en étuve et après calcination, par exemple pendant deux heures à environ 500°C, au moins un élément, par exemple le platine, est déposé, par exemple par échange anionique avec de l'acide hexachloroplatinique en présence d'un agent compétiteur (par exemple l'acide chlorhydrique), ledit dépôt étant suivi d'une calcination par exemple pendant environ 2 heures à environ 500°C.

Le platine est généralement introduit dans la matrice sous forme d'acide hexachloroplatinique, mais pour tout métal noble peuvent être également utilisés des composés ammoniaqués ou des composés tels que par exemple le chloroplatinate d'ammonium, le dichlorure de platine dicarbonyle, l'acide hexahydroxyplatinique, le chlorure de palladium ou le nitrate de palladium.

L'emploi dans la présente invention d'au moins un métal noble de la famille du platine peut à titre d'exemple se faire grâce à l'utilisation de composés ammoniaqués. Dans ce cas, le métal noble sera déposé dans la zéolithe.

Dans le cas du platine, on peut citer par exemple les sels de platine II tétramines de formule $Pt(NH_3)_4X_2$ ; les sels de platine IV hexamines de formule $Pt(NH_3)_6X_4$ ; les sels de platine IV halogénopentamines de formule $(PtX(NH_3)_5)X_3$ ; les sels de platine N tétrahalogénodiamines de formule $PtX_4(NH_3)_2$ ; les complexes de platine avec les halogènes-polycétones et les composés halogénés de formule $H(Pt(acac)_2X)$ ; X étant un halogène choisi dans le groupe formé par le chlore, le fluor, le brome et l'iode et de préférence X étant le chlore, et acac représentant le groupe $C_5H_7O_2$ dérivé de l'acétylacétone.

L'introduction du métal noble de la famille du platine est de préférence effectuée par imprégnation à l'aide d'une solution aqueuse ou organique de l'un des composés organométalliques cités ci-dessus. Parmi les solvants organiques utilisables, on peut citer les hydrocarbures paraffiniques, naphténiques ou aromatiques, et les composés organiques halogénés ayant par exemple de 1 à 12 atomes de carbone par molécule. On peut citer par exemple le n-heptane, le méthylcyclohexane, le toluène et le chloroforme. On peut aussi utiliser les mélanges de solvants.

Le métal additionnel, éventuellement introduit en plus, choisi dans le groupe formé par les éléments des groupes IB, IIB, IIIA, IVA, VIB et VIIB, peut être introduit par l'intermédiaire de composés tels que par exemple les chlorures, les bromures et les nitrates, les alkyls d'éléments des groupes IB, IIB, IIIA, IVA, VIB et VIIB, soit par exemple l'étain et l'indium, les alkyl étain, le nitrate et le chlorure d'indium. Ce métal peut également être introduit sous la forme d'au moins un composé organique choisi dans le groupe constitué par les complexes dudit métal, en particulier les complexes polycétoniques du métal et les hydrocarbylmétaux tels que les alkyles, les cycloalkyles, les aryles, les alkylaryles et les arylalkyles métaux. Dans ce dernier cas, l'introduction du métal est avantageusement effectuée à l'aide d'une solution du composé organométallique dudit métal dans un solvant organique. On peut également employer des composés organohalogénés du métal. Comme composés du métal, on peut citer en particulier le tétrabutylétain dans le cas de l'étain, et le triphénylindium dans le cas de l'indium.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer par exemple le n-heptane, le méthylcyclohexane et le chloroforme. On peut aussi utiliser des mélanges des solvants définis ci-dessus.

On peut aussi envisager d'introduire au moins un métal choisi dans le groupe formé par les éléments des groupes IB, IIB, IIIA, IVA, VIB et VIIB. Ce métal additionnel peut être éventuellement introduit à tout moment de la préparation, de préférence préalablement au dépôt d'un ou plusieurs métaux du groupe VIII. Si ce métal est introduit avant le métal noble, le composé du métal utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal par exemple le tétrabutylétain. Dans ce cas, avant de procéder à l'introduction d'au moins un métal noble, on procède à une calcination sous air.

La mise en forme du catalyseur selon l'invention est généralement telle que le catalyseur est de préférence sous forme d'extrudés ou de billes en vue de son utilisation.

La préparation du catalyseur se termine généralement par une calcination, habituellement à une température comprise entre environ 250°C et 600°C, bornes incluses, pour une durée d'environ 0,5 à 10 heures, de préférence précédée d'un séchage, par exemple à l'étuve, à une température comprise entre la température ambiante et 250°C, de préférence entre 40 et 200°C. Ladite étape de séchage est de préférence menée pendant la montée en température nécessaire pour effectuer ladite calcination.

Dans le cas où le catalyseur de la présente invention contient du soufre, le soufre est introduit sur le catalyseur mis en forme, calciné, contenant le ou les métaux cités précédemment, soit *in-situ* avant la réaction catalytique, soit *ex-situ*. La sulfuration éventuelle intervient après la réduction. Dans le cas d'une sulfuration in situ, la réduction, si le catalyseur n'a pas été préalablement réduit, intervient avant la sulfuration. Dans le cas d'une sulfuration ex-situ, on effectue la réduction puis la sulfuration. La sulfuration s'effectue en présence d'hydrogène en utilisant tout agent sulfurant bien connu de l'homme de métier, tel que par exemple le sulfure de diméthyle ou le sulfure d'hydrogène. Par exemple, le catalyseur est traité avec une charge contenant du sulfure de diméthyle en présence d'hydrogène, avec une concentration telle que le rapport atomique soufre/métal soit de 1,5. Le catalyseur est ensuite maintenu pendant environ 3 heures à environ 400°C sous débit d'hydrogène avant l'injection de la charge.

Le catalyseur à base de zéolithe EUO préparée selon le procédé de la présente invention est mis en oeuvre dans les réactions d'isomérisation d'une coupe C8 aromatique, comprenant par exemple soit un mélange de xylènes, soit de l'éthylbenzène, soit un mélange de xylène(s) et, d'éthylbenzène. Ledit procédé est mis en oeuvre généralement selon les conditions opératoires suivantes :

- une température comprise entre 300°C et 500°C, de préférence entre 320°C et 450°C et de manière encore plus préférée entre 340°C et 430°C,
- une pression partielle d'hydrogène comprise entre 0,3 et 1,5 MPa, de préférence entre 0,4 et 1,2 MPa, et de manière encore préférée entre 0,7 et 1,2 MPa,
- une pression totale comprise entre 0,45 et 1,9 Mpa, de préférence entre 0.6 et 1,5 MPa,
- une vitesse spatiale d'alimentation, exprimée en kilogramme de charge introduite par kilogramme de catalyseur et par heure, comprise entre 0,25 et $30h^{-1}$, de préférence entre 1 et $10h^{-1}$, et de manière encore plus préférée entre 2 et 6 $h^{-1}$.

Les exemples suivants illustrent l'invention.

EXEMPLES 1 à 17 :

Synthèse de zéolithe EU-1 (ou TPZ-3) de rapport Si/Al égal à 15 avec le bromure d'hexaméthonium comme structurant organique et des germes de zéolithe de structure et/ou de rapport Si/Al différents, ayant un rapport Si/Al < 200.

Les exemples 1 et 2 correspondent à des synthèses réalisées sans ajout de germes pour comparaison. Les exemples 3 à 17 correspondent à des synthèses réalisées avec les germes de zéolithe dont les caractéristiques sont reportées ci-après.

Les zéolithes ajoutées en tant que germes se présentent sous différentes formes cationiques (Na, K, $NH_4$, $NH_4$+TMA, Na+Hx, H) et sous formes de particules de taille variable (de 3 à 115 µm pour le diamètre médian Dv,50). Ces germes sont différents du solide EU-1 synthétisé au niveau du type structural et/ou du rapport Si/Al.

Le mélange de synthèse a la composition suivante :

| | |
|---|---|
| $SiO_2$ (mol) | 60 |
| $Al_2O_3$ (mol) | 2 |
| $Na_2O$ (mol) | 10 |
| $HxBr_2$ (mol) | 20 |
| $H_2O$ (mol) | 2800 |
| germes/$SiO_2$ (g/g) | 0 (ex. 1 et 2) ou 0,02 (ex. 3 à 16) |
| $Hx\,Br_2$ = bromure d'hexaméthonium = $Me_3\,N\,(CH_2)_6\,N\,Me_3{}^{2+}(Br^-)_2$ | |

| | | germes | | | | | |
|---|---|---|---|---|---|---|---|
| Exemples | zéolithe | type structural | Si/Al (mol/mol) | forme (cations) | Dv,10 (µm) | Dv,50 (µm) | Dv,90 (µm) |
| 3 (invention) | ZSM-5 | MFI | 14,3 | Na | 48 | 115 | 205 |
| 4 (invention) | ZSM-5 | MFI | 27,7 | H | 52 | 107 | 173 |
| 5 (invention) | ZSM-5 | MFI | 76,5 | H | 2,8 | 5,8 | 24 |
| 6 (invention) | ZSM-5 | MFI | 150 | H | 1,7 | 4,8 | 14 |
| 7 (comparatif) | silicalite | MFI | >500 | - | 1,4 | 2,7 | 5,2 |
| 8 (invention) | A | LTA | 1,0 | K | 1,1 | 5,3 | 11 |
| 9 (invention) | X | FAU | 1,3 | Na | 1,3 | 3,4 | 8,7 |

(suite)

| Exemples | zéolithe | type structural | Si/Al (mol/mol) | forme (cations) | Dv,10 (µm) | Dv,50 (µm) | Dv,90 (µm) |
|---|---|---|---|---|---|---|---|
| | | | | germes | | | |
| 10 (invention) | Y | FAU | 2,8 | NH$_4$ | 1,4 | 3,1 | 7,0 |
| 11 (invention) | mordénite | MOR | 118 | H | 5,1 | 15 | 26 |
| 12 (invention) | mordénite | MOR | 5 | NH4$_4$ | 5,1 | 15 | 26 |
| 13 (invention) | mazzite | MAZ | 3,6 | NH$_4$+TMA | 7,0 | 18 | 25 |
| 14 (invention) | EU-1 | EUO | 25,2 | NH$_4$ | 3,0 | 16 | 26 |
| 15 (invention) | EU-1 | EUO | 69,7 | Na+Hx | 3,0 | 16 | 40 |
| 16 (invention) | EU-1 | EUO | 144 | Na+Hx | 3,2 | 17 | 33 |
| 17 (comparatif) | EU-1 | EUO | >500 | Na-Hx | 1,5 | 2,9 | 5,8 |

TMA = tétraméthylammonium

Hx = hexaméthonium

Dv,X = diamètre de la sphère équivalente des particules tel que X % volume des particules ont une taille inférieure audit diamètre

On prépare la solution A composée de silicium et de structurant en diluant 12,03 g de bromure d'hexaméthonium (Fluka, 97 %) dans 57,42 g d'eau puis en ajoutant 14,50 g de sol de silice colloïdal (Ludox HS40, Dupont, 40 % SiO$_2$). On dissout ensuite 0,985 g d'hydroxyde de sodium solide (Prolabo, 99 %) et 0,715 g d'aluminate de sodium solide (Prolabo, 46 % Al$_2$O$_3$, 33 % Na$_2$O) dans 7,18 g d'eau pour former la solution B. On ajoute la solution B dans la solution A sous agitation puis 7,18 g d'eau. On mélange jusqu'à homogénéisation et on ajoute 0,116 g (exemples 3 à 17) de germes de zéolithes séchées (pas d'ajout de germes pour les exemples 1 et 2). On fait réagir le mélange résultant dans un autoclave de 125 ml sous agitation à 180°C sous la pression autogène. Après refroidissement, on filtre le produit et on le lave avec 0,5 litre d'eau déminéralisée puis on le sèche en étuve ventilée à 120°C.

Les résultats de diffraction des rayons X et d'analyse chimique sont reportés dans le tableau 1 en fonction de l'ajout de germes ou pas et des différentes zéolithes ajoutées en tant que germes. L'utilisation des différentes zéolithes à rapport Si/Al inférieur à 200 en tant que germes (exemples 3 à 6 et 8 à 16) conduit à la zéolithe EU-1 pure (cristallinité de 100 % ±3, rapport Si/Al voisin de 15), de rendement maximal (environ 5 %) en 96 heures à 180°C. Dans des conditions identiques, les essais avec des germes de zéolithe de rapport Si/Al supérieur à 200 donnent un solide non complètement cristallisé en zéolithe EU-1 (exemples 7 et 17). Dans les conditions identiques, l'expérience sans addition de germes (exemple 2) ne produit pas la zéolithe EU-1. Ainsi, dans l'exemple 1, il faut aller jusqu'à 125 heures de durée de synthèse pour permettre la formation de la zéolithe EU-1 pure et de rendement maximal.

EXEMPLES 18 à 33:

Synthèse de zéolithe ZSM-50 de rapport Si/Al égal à 125 avec les précurseurs du chlorure de dibenzyldiméthylammonium comme structurant organique et des germes de zéolithe de structure et/ou de rapport Si/Al différents, ayant un rapport Si/Al<200.

Les exemples 18 et 19 correspondent à des synthèses réalisées sans ajout de germes pour comparaison. Les exemples 20 à 33 correspondent à des synthèses réalisées avec les germes de zéolithes identiques à celles utilisées dans les exemples précédents.

Le mélange de synthèse a la composition suivante :

| SiO$_2$ (mol) | 60 |
|---|---|
| Al$_2$O$_3$ (mol) | 0,24 |
| Na$_2$O (mol) | 3,6 |
| BDMA (mol) | 6 |
| BCl (mol) | 6 |
| H$_2$O (mol) | 1000 |
| germes/SiO$_2$ (g/g) | 0 (ex. 18 et 19) ou 0,02 (ex. 20 à 33) |
| BDMA = benzyidiméthylamine | |
| BCI = chlorure de benzyle | |

On prépare la solution C composée de silicium et des précurseurs de structurant en diluant 3,537 g de benzyldiméthylamine (Fluka, 98 %) et 3,274 g de chlorure de benzyle (Fluka, 99 %) dans 42,72 g d'eau puis en ajoutant 38,43 de sol de silice colloïdal (Ludox HS40, Dupont, 40 % SiO$_2$). On dissout ensuite 1,133 g d'hydroxyde de sodium solide (Prolabo, 99 %) et 0,227 g d'aluminate de sodium solide (Prolabo, 46 % Al$_2$O$_3$, 33 % Na$_2$O) dans 5,34 g d'eau pour former la solution D. On ajoute la solution D dans la solution C sous agitation puis 5,34 g d'eau. On mélange jusqu'à homogénéisation et on ajoute 0,307 g (exemples 20 à 33) de germes de zéolithes séchées (pas d'ajout de germes pour les exemples 18 et 19). On fait réagir le mélange résultant dans un autoclave de 125 ml sous agitation pendant 75-98 heures à 160°C sous pression autogène. Après refroidissement, on filtre le produit et on le lave avec 1,5 litre d'eau déminéralisée puis on le sèche en étuve ventilée à 120°C.

Les résultats de diffraction des rayons X et d'analyse chimique sont reportés dans le tableau 2 en fonction de l'ajout de germes ou pas et des différentes zéolithes ajoutées en tant que germes. L'utilisation des différentes zéolithes à rapport Si/Al inférieur à 200 en tant que germes (exemples 20 à 23 et 25 à 32) conduit aux zéolithes EU-1, ZSM-50 pures (cristallinité de 100 % ± 3, rapport Si/Al voisin de 125), de rendement maximal (environ 15 %) en 75 heures à 160°C.

Dans des conditions identiques, les essais avec des germes de zéolithe de rapport Si/Al élevé donnent un solide non complètement cristallisé en zéolithe EU-1 et ZSM-50 (exemples 24 et 33). Dans les conditions identiques, l'expérience sans addition de germes (exemples 2 et 19) ne produit pas la zéolithe EU-1 ni la zéolithe ZSM-50. Ainsi, dans l'exemple 1 et l'exemple 18, il faut aller jusqu'à 125 heures ou 98 heures de durée de synthèse pour permettre la formation de la zéolithe EU-1 et de la zéolithe ZSM-50 pures et de rendement maximal.

Tableau 1 : synthèses des zéolithes EU-1 ou TPZ-3 de rapport Si/Al voisin de 15 avec des germes de zéolithes de divers types structuraux et rapports Si/Al

| n° exemple | 1 (compa ratif) | 2 (compa ratif) | 3 | 4 | 5 | 6 | 7 (compa ratif) | 8 | 9 | 10 | 11 | 12 | 13 | 14 | 15 | 16 | 17 (compa ratif) |
|---|---|---|---|---|---|---|---|---|---|---|---|---|---|---|---|---|---|
| **Gel** | | | | | | | | | | | | | | | | | |
| formulat. | 60 SiO$_2$ - 2 Al$_2$O$_3$ - 10 Na$_2$O - 20 HxBr$_2$ - 2800 H$_2$O - 2 % germes | | | | | | | | | | | | | | | | |
| germes | sans | sans | Na-ZSM-5 Si/Al =14,3 | H-ZSM-5 Si/Al =25,7 | H-ZSM-5 Si/Al =76,5 | H-ZSM-5 Si/Al =150 | silica-lite Si/Al >500 | K-A Si/Al =1,0 | Na-X Si/Al =1,3 | NH4-Y Si/Al =2,8 | H-MOR Si/Al= 118 | NH4-MOR Si/Al =5 | NH4-TMA-MAZ Si/Al= =3,6 | Na-Hx-EU-1 Si/Al =25,2 | Na-Hx-EU-1 Si/Al =69,7 | Na-Hx-EU-1 Si/Al =144 | Na-Hx-EU-1 Si/Al >500 |
| **Cristallisation** | | | | | | | | | | | | | | | | | |
| Température (°C) | 180 | | | | | | | | | | | | | | | | |
| Temps (h) | 125 | 96 | | | | | | | | | | | | | | | |
| **Solide** | | | | | | | | | | | | | | | | | |
| Phase (DRX) | 100 % EU-1 | AMO | 101 % EU-1 | 98 % EU-1 | 97 % EU-1 | 99 % EU-1 | 85 % EU-1+ AMO | 102 % EU-1 | 100 % EU-1 | 101 % EU-1 | 99 % EU-1 | 100% EU-1 | 99 % EU-1 | 100 % EU-1 | 102 % EU-1 | 103 % EU-1 | 90 % EU-1+ AMO |
| Si/Al (FX) | 15,4 | nd | 13,8 | 14,2 | 13,8 | 14,5 | nd | 13,7 | 14,9 | 14,7 | 13,9 | 15,1 | 13,8 | 13,9 | 14,1 | 13,8 | nd |
| Rendement en solide (%) | 5,3 | 6,1 | 5,1 | 5,4 | 5,2 | 4,9 | 5,1 | 5,7 | 5,1 | 5,4 | 5,3 | 5,1 | 5,6 | 5,2 | 5,6 | 5,1 | 5,7 |

AMO = amorphe  
Hx = hexaméthonium  
FX= fluorescence X

TMA = tétraméthylammonium  
DRX = Diffraction des rayons X, avec pour référence l'exemple 1

Tableau 2 : synthèses de la zéolithe ZSM-50 de rapport Si/Al voisin de 125 avec des germes de zéolithes de divers types structuraux et rapports Si/Al

| n° exemple | 18 (comp aratif) | 19 (comp aratif) | 20 | 21 | 22 | 23 | 24 (comp aratif) | 25 | 26 | 27 | 28 | 29 | 30 | 31 | 32 | 33 |
|---|---|---|---|---|---|---|---|---|---|---|---|---|---|---|---|---|
| **Gel** | | | | | | | | | | | | | | | | |
| Formulat. | $60\ SiO_2 - 0{,}3\ Al_2O_3 - 10\ Na_2O - 20\ HxBr_2 - 2800\ H_2O - 2\ \%\ germes$ | | | | | | | | | | | | | | | |
| Germes | sans | sans | Na-ZSM-5 Si/Al =14,3 | H-ZSM-5 Si/Al =25,7 | H-ZSM-5 Si/Al =76,5 | H-ZSM-5 Si/Al =150 | silica-lite Si/Al >500 | K-A Si/Al=1 | Na-X Si/Al =1,3 | NH4-Y Si/Al =2,8 | NH4-MOR Si/Al= 118 | NH4-TMA-MAZ Si/Al= 3,6 | Na-Hx-EU-1 Si/Al =25,2 | Na-Hx-EU-1 Si/Al =69,7 | Na-Hx-EU-1 Si/Al =144 | Na-Hx-EU-1 Si/Al >500 |
| **Cristallisation** | | | | | | | | | | | | | | | | |
| Température (°C) | 160 | | | | | | | | | | | | | | | |
| Temps (h) | 98 | 75 | | | | | | | | | | | | | | |
| **Solide** | | | | | | | | | | | | | | | | |
| Phase (DRX) | 100 % ZSM-50 | AMO | 97 % ZSM-50 | 99 % ZSM-50 | 99 % ZSM-50 | 100 % ZSM-50 | 75 % ZSM-50+ AMO | 101 % ZSM-50 | 103 % ZSM-50 | 98 % ZSM-50 | 101 % ZSM-50 | 98 % ZSM-50 | 100 % ZSM-50 | 102 % ZSM-50 | 98 % ZSM-50 | 85 % ZSM-50+ AMO |
| Si/Al (FX) | 133 | nd | 139 | 122 | 118 | 135 | nd | 109 | 113 | 123 | 132 | 121 | 131 | 146 | 137 | nd |
| Rendement en solide (%) | 15,5 | 17,1 | 15,2 | 16,0 | 15,3 | 15,8 | 15,6 | 15,5 | 15,2 | 15,0 | 15,8 | 18,9 | 15,6 | 15,0 | 14,8 | 16,2 |

AMO = amorphe  
TMA = tétraméthylammonium  
Hx = hexaméthonium  

BDMA = benzyldiméthylamine  
BCl chlorure de benzyl  

DRX = Diffraction des rayons X, avec comme référence l'exemple 17  
FX= fluorescence X

EP 0 999 182 B1

***Exemple 34 :*** Préparation du catalyseur A non conforme à l'invention, contenant la zéolithe EU-1 synthétisée en l'absence de germes telle que décrite dans l'exemple 1 et 0 ,3 % poids de platine.

La matière première utilisée est une zéolithe EU-1 brute de synthèse, comprenant le structurant organique, du silicium et de l'aluminium, possédant un rapport atomique Si/Al global égal à 15,4 , une teneur pondérale en sodium par rapport au poids en zéolithe EU-1 sèche d'environ 1,5 %.
Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange.
A l'issue de ces traitements, la zéolithe EU-1 sous forme $NH_4$ a un rapport Si/Al atomique global égal à 19,9 , une teneur pondérale en sodium par rapport au poids de zéolithe EU-1 sèche de 55 ppm.
La zéolithe EU-1 est ensuite mise en forme par extrusion avec un gel d'alumine de manière à obtenir, après séchage et calcination sous air sec, un support S1 constitué d'extrudés de 1,4 mm de diamètre, qui contient en poids 10 % de zéolithe EU-1 sous forme H et 90 % d'alumine.
Le support S1 ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous un débit d'air sec à la température de 500°C pendant une heure.
Le catalyseur A ainsi obtenu contient en poids, 10,0 % de zéolithe EU-1 forme hydrogène, 89,7 % d'alumine et 0,3 % de platine.

***Exemple 35 :*** Préparation du catalyseur B conforme à l'invention, contenant la zéolithe EU-1 synthétisée en présence de germes $NH_4$-Y telle que décrite dans l'exemple 10 et 0,3 % poids de platine.

La matière première utilisée est la zéolithe EU-1 brute de synthèse, comprenant le structurant organique, du silicium et de l'aluminium, possédant un rapport atomique Si/Al global égal à 14,7 , une teneur pondérale en sodium par rapport au poids en zéolithe EU-1 sèche d'environ 1,4 %.
Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange.
A l'issue de ces traitements, la zéolithe EU-1 sous forme $NH_4$ a un rapport Si/Al atomique global égal à 19,2, une teneur pondérale en sodium par rapport au poids de zéolithe EU-1 sèche de 30 ppm.
La zéolithe EU-1 est ensuite mise en forme par extrusion avec un gel d'alumine de manière à obtenir, après séchage et calcination sous air sec, un support S2 constitué d'extrudés de 1,4 mm de diamètre, qui contient en poids 10 % de zéolithe EU-1 sous forme H et 90 % d'alumine.
Le support S2 ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous un débit d'air sec à la température de 500°C pendant une heure.
Le catalyseur B ainsi obtenu contient en poids, 10,0 % de zéolithe EU-1 forme hydrogène, 89,7 % d'alumine et 0,3 % de platine.

***Exemple 36 :*** Préparation du catalyseur C conforme à l'invention, contenant la zéolithe EU-1 synthétisée en présence de germes MOR (Si/Al=5) telle que décrite dans l'exemple 12 et 0,3 % poids de platine.

La matière première utilisée est la zéolithe EU-1 brute de synthèse, comprenant le structurant organique, du silicium et de l'aluminium, possédant un rapport atomique Si/Al global égal à 13,9 , une teneur pondérale en sodium par rapport au poids en zéolithe EU-1 sèche d'environ 1,5.
Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange.
A l'issue de ces traitements, la zéolithe EU-1 sous forme $NH_4$ a un rapport Si/Al atomique global égal à 18,5, une teneur pondérale en sodium par rapport au poids de zéolithe EU-1 sèche de 45 ppm.
La zéolithe EU-1 est ensuite mise en forme par extrusion avec un gel d'alumine de manière à obtenir, après séchage et calcination sous air sec, un support S3 constitué d'extrudés de 1,4 mm de diamètre, qui contient en poids 10 % de zéolithe EU-1 sous forme H et 90 % d'alumine.
Le support S3 ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous un débit d'air sec à la température de 500°C pendant une heure.

Le catalyseur C ainsi obtenu contient en poids, 10,0 % de zéolithe EU-1 forme hydrogène, 89,7 % d'alumine et 0,3 % de platine.

***Exemple 37 :*** Préparation du catalyseur D non conforme à l'invention, contenant la zéolithe EU-1 synthétisée en présence de germes de silicalite (Si/Al>500) telle que décrite dans l'exemple 7 et 0,3 % poids de platine.

La matière première utilisée est la zéolithe EU-1 brute de synthèse, non complètement cristallisée (85% EU-1+amorphe) et dont le rapport Si/Al n'a pu être déterminé. La teneur pondérale en sodium par rapport au poids en zéolithe EU-1 sèche d'environ 1,5%.

Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange.

A l'issue de ces traitements, la zéolithe EU-1 sous forme $NH_4$ a un rapport Si/Al atomique global égal à 18,5, une teneur pondérale en sodium par rapport au poids de zéolithe EU-1 sèche de 40 ppm.

La zéolithe EU-1 est ensuite mise en forme par extrusion avec un gel d'alumine de manière à obtenir, après séchage et calcination sous air sec, un support S4 constitué d'extrudés de 1,4 mm de diamètre, qui contient en poids 10 % de zéolithe EU-1 sous forme H et 90 % d'alumine.

Le support S3 ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous un débit d'air sec à la température de 500°C pendant une heure.

Le catalyseur D ainsi obtenu contient en poids, 10,0 % de zéolithe EU-1 forme hydrogène, 89,7 % d'alumine et 0,3 % de platine.

***Exemple 38 :*** Evaluation des propriétés catalytiques des catalyseurs A, B, C et D en isomérisation d'une coupe C8 aromatique.

Les performances des catalyseurs A, B, C et D ont été évaluées dans l'isomérisation d'une coupe C8 aromatique contenant principalement du méta-xylène, de l'orthoxylène et de l'éthylbenzène. Les conditions opératoires sont les suivantes :

- température : 390°C,
- pression totale : 15 bar, (1 bar = 0,1 MPa)
- pression partielle d'hydrogène : 12 bar.

Les tests sont effectués en laboratoire, sur 5 g de catalyseur et sans recyclage d'hydrogène.

Les catalyseurs sont préalablement traités avec une charge contenant du disulfure de diméthyle (DMDS) en présence d'hydrogène, avec une concentration telle que le rapport atomique soufre/métal soit de 1,5. Le catalyseur est ensuite maintenu pendant 3 heures à 400°C sous débit d'hydrogène, puis la charge est injectée.

Les catalyseurs ont été comparés en terme d'activité (par les approches à l'équilibre du para-xylène et de l'éthylbenzène, et par la conversion de l'éthylbenzène) et en terme de sélectivité (par les pertes nettes à iso-approche à l'équilibre du para-xylène).

Les réactions parasites conduisent à trois types de pertes : les pertes vers les paraffines résultant essentiellement de réactions d'ouverture de cycles naphténiques suivies de craquage, les pertes vers les aromatiques formés par réactions de dismutation et de transalkylation des aromatiques à 8 atomes de carbone (AC8), et enfin les pertes vers les naphtènes dont les naphtènes à 8 atomes de carbone (N8) dus à l'hydrogénation des aromatiques. Les N8 pouvant être recyclés, on comparera les pertes par craquage et dismutation/transalkylation incluant les naphtènes autres que N8 (dont la somme constitue les pertes nettes). Pour le calcul des approches à l'équilibre (AEQ), les concentrations en para-xylène (%pX) sont exprimées par rapport aux trois isomères xylènes.

L'approche à l'équilibre (AEQ) est définie de la manière suivante :

$$pX\ AEQ\ (\%) = 100 \times (\%pX_{effluent} - \%pX_{charge}) / (\%pX_{équilibre} - \%PX_{charge})$$

Les pertes par craquage (P1) sont des pertes en AC8 sous forme de paraffines (PAR) de C1 à C8 :

$$P1(\%poids) = 100 \times [(\%PAR_{effluent} \times poids \text{ d'effluent}) - (\%PAR_{charge} \times poids \text{ de}$$

charge)] / (%AC8$_{charge}$xpoids de charge)

Les pertes par dismutation/transalkylation (P2) sont des pertes en AC8 sous forme de naphtènes autres que N8, de toluène, de benzène et de C9+ aromatiques (OAN) :

$$P2(\%poids) = 100 \times [(\%OAN_{effluent} \times poids \text{ d'effluent}) - (\%OAN_{charge} \times poids \text{ de}$$

charge)] / (%AC8$_{charge}$xpoids de charge)

La somme des pertes P1 et P2 représente les pertes nettes.

Les données présentées dans le tableau 3 ont été obtenues à iso-conditions expérimentales.

Tableau 3

| Catalyseur | A (non conforme) | B (conforme) | C (conforme) | D (non conforme) |
|---|---|---|---|---|
| pX AEQ (%) | 97,8 | 98,0 | 98,2 | 85,2 |
| conversion EB (%) | 59,5 | 59,3 | 59,8 | 36,2 |

On constate d'après les résultats du tableau 3 que les catalyseurs B et C conformes à l'invention conduisent à des résultats comparables à ceux obtenus avec le catalyseur A non conforme, et que le catalyseur D non conforme est beaucoup moins actif que les trois autres catalyseurs.
Par ailleurs, ces catalyseurs ont été comparés à pX AEQ plus faible (environ 95,5 %) en faisant varier les débits massiques de charge. Ces résultats sont présentés dans le tableau 4.

Tableau 4

| Catalyseurs | A (non conforme) | B (conforme) | C (conforme) | D (non conforme) |
|---|---|---|---|---|
| pX AEQ (%) | 95,3 | 95,5 | 95,1 | 95,1 |
| pertes nettes (% poids) | 4,9 | 5,1 | 4,7 | 4,8 |

A iso pX AEQ de 95,5 % environ, le tableau 4 montre que les quatre catalyseurs sont tous aussi sélectifs.
L'activité et la sélectivité obtenues lors de l'utilisation des catalyseurs B et C, à base de zéolithes de structure EUO, obtenues respectivement en utilisant des germes NH$_4$-Y et MOR, en isomérisation d'une coupe C8 aromatique, sont donc comparables à celles d'un catalyseur contenant une zéolithe de structure EUO de rapport Si/Al proche et obtenue selon un mode de synthèse ne faisant pas intervenir de germes et décrit dans l'art antérieur. La catalyseur D obtenu en utilisant des germes non conformes à l'invention (Si/Al>500) est nettement moins actif que les autres.
Enfin, les catalyseurs A, B, C et D ont été comparés en terme de stabilité temporelle toujours dans les conditions expérimentales décrites au début de cet exemple et sur une durée de 400 heures.
La stabilité est évaluée à partir de l'évolution de la conversion de l'éthylbenzène. Les résultats sont présentés dans le tableau 5.

Tableau 5

| Catalyseur | A (non conforme) | B (conforme) | C (conforme) | D (non conforme) |
|---|---|---|---|---|
| Conversion EB (%) à t=36 h | 59,5 | 59,3 | 59,8 | 36,2 |
| Conversion EB (%) à t=400 h | 54,4 | 53,9 | 53,8 | 35,1 |
| chute de conversion EB (%) | 3,2 | 3,4 | 3,1 | 3,1 |

On constate que la désactivation des quatre catalyseurs A, B, C et D sur 400 heures est comparable.
Les propriétés catalytiques (activité, sélectivité et stabilité) des trois catalyseurs A, B et C sont comparables. L'introduction de ce nouveau mode de synthèse de la zéolithe de type structural EUO dans la préparation de ce catalyseur d'isomérisation des coupes C8 aromatiques est donc d'un apport tout à fait notable en terme de coût puisque le temps de synthèse de la zéolithe est réduit d'environ 25 % tout en conservant les propriétés catalytiques du catalyseur selon l'invention. L'utilisation de germes de zéolithes de rapport Si/Al<200 est indispensable à l'obtention d'une zéolithe de type structural EUO conduisant à un bon catalyseur. En effet, le catalyseur D présente une activité nettement plus faible.

## Revendications

1. Procédé de préparation d'un matériau zéolithique de type structural EUO comprenant le mélange en milieu aqueux d'au moins une source d'au moins un élément X choisi parmi le silicium et le germanium, d'au moins une source d'au moins un élément T choisi parmi le fer, l'aluminium, le gallium, bore, le titane, le vanadium, le zirconium, le molybdène, l'arsenic, l'antimoine, le chrome et le manganèse, d'au moins une source d'un structurant organique, **caractérisé en ce qu'**on utilise des germes d'au moins un matériau zéolithique comprenant au moins un élément X' choisi parmi le silicium et le germanium et au moins un élément T choisi parmi le fer, l'aluminium, le gallium, le bore, le titane, le vanadium, le zirconium, le molybdène, l'arsenic, l'antimoine, le chrome et le manganèse, de rapport X'/T' inférieur à 200, lesdits germes étant différents de la zéolithe de type structural EUO que l'on prépare.

2. Procédé selon la revendication 1 dans lequel les germes de matériau zéolithique ont un type structural différent du type structural EUO.

3. Procédé selon l'une des revendications 1 à 2 tel que les germes ont une composition chimique de la charpente cristalline différente de la composition chimique de la charpente cristalline de la zéolithe de type structural EUO que l'on prépare.

4. Procédé selon l'une des revendications 1 à 3 tel que le matériau zéolithique utilisé comme germes est choisi parmi les zéolithes de type structural LTA telle la zéolithe A, de type structural LTL, de type structural FAU telles les zéolithes X et Y, de type structural MOR, de type structural MAZ, de type structural OFF, de type structural FER, de type structural ERI, de type structural BEA, de type structural MFI telles la ZSM-5 et la silicalite, de type structural MTW, de type structural MTT, de type structural LEV, de type structural TON, de type structural NES telle la zéolithe NU-87, les zéolithes NU-85, NU-86, NU-88, IM-5, ou les zéolithes de type structural EUO de composition chimique de la charpente cristalline différente de la zéolithe EUO que l'on prépare telles les zéolithes EU-1, ZSM-50 et TPZ-3.

5. Procédé selon l'une des revendications 1 à 4 tel que les germes utilisés sont choisis parmi des germes d'au moins un matériau zéolithique de type structural LTA, FAU, MOR, MFI et EUO.

6. Procédé selon l'une des revendications 1 à 5 dans lequel les germes de matériau zéolithique sont introduits à n'importe quel moment de la préparation.

7. Procédé selon l'une des revendications 1 à 6 dans lequel les germes de matériau zéolithique sont introduits après l'homogénéisation au moins en partie du mélange aqueux contenant la source d'élément X, la source d'élément T, la source de structurant organique.

8. Procédé selon l'une des revendications 1 à 7 tel que l'élément X est le silicium et l'élément T est l'aluminium.

9. Procédé selon l'une des revendications 1 à 8 tel que le structurant organique est un dérivé alkylé de polyméthylène $\alpha$-$\omega$ diammonium de formule : $R_1R_2R_3\,N^+\,(CH_2)_n\,N^+\,R_4R_5R_6$ et/ou un produit de dégradation d'amine correspondant audit dérivé et/ou un précurseur correspondant audit dérivé, n étant compris entre 3 et 12 et $R_1$ à $R_6$, identiques ou différents, pouvant représenter des radicaux alkyles ou hydroxyalkyles ayant de 1 à 8 atomes de carbone, jusqu'à cinq des radicaux $R_1$ à $R_6$ pouvant être de l'hydrogène.

10. Procédé selon l'une des revendications 1 à 8 tel que le structurant organique est un composé comprenant du dibenzyldiméthylammonium et/ou un produit de dégradation d'amine correspondant audit dérivé et/ou un précurseur correspondant audit dérivé.

11. Procédé selon l'une des revendications 1 à 10 tel qu'on introduit au moins un sel P de métal alcalin ou d'ammonium.

12. Procédé selon l'une des revendications 1 à 9 dans lequel le mélange réactionnel a la composition suivante, exprimée sous forme d'oxydes :

| | |
|---|---|
| $XO_2/T_2O_3$ (mol/mol) | au moins 10 |
| $OH^-/XO_2$ (mol/mol) | 0,002 à 2,0 |
| $Q/XO_2$ (mol/mol) | 0,002 à 2 |
| $Q/(M^+ + Q)$ (mol/mol) | 0,1 à 1,0 |
| $H_2O/XO_2$ (mol/mol) | 1 à 500 |
| $P/XO_2$ (mol/mol) | 0 à 5 |
| $S/XO_2$ (g/g) | 0,0001 à 0,1 |

où

X est le silicium et/ou le germanium,

T est au moins un élément choisi parmi l'aluminium, le fer, le gallium, le bore, le titane, le vanadium, le zirconium, le molybdène, l'arsenic, l'antimoine, le chrome et le manganèse

$M^+$ représente un métal alcalin ou l'ion ammonium,

Q représente le structurant organique, ou les produits de décomposition correspondant audit dérivé ou les précurseurs dudit dérivé,

S représente les germes de zéolithe présents sous la forme brute, séchée, calcinée ou échangée, avec un rapport X'/T' inférieur à 200.

P représente le sel de métal alcalin ou d'ammonium.

**Patentansprüche**

1. Verfahren zur Herstellung eines zeolithischen Materials vom Strukturtyp EUO, umfassend das Mischen in einem wässrigen Milieu von wenigstens einer Quelle wenigstens eines Elements X, gewählt unter Silizium und Germanium, wenigstens einer Quelle wenigstens eines Elements T, gewählt unter Eisen, Aluminium, Gallium, Bor, Titan, Vanadium, Zirkonium, Molybdän, Arsen, Antimon, Chrom und Mangan, wenigstens einer Quelle eines organischen Strukturbildners, **dadurch gekennzeichnet dass** man Keime wenigstens eines zeolithischen Materials verwendet, das wenigstens ein Element X' umfasst, gewählt unter Silizium und Germanium und wenigstens ein Element T' umfasst, gewählt unter Eisen, Aluminium, Gallium, Bor, Titan, Vanadium, Zirkonium, Molybdän, Arsen, Antimon, Chrom und Mangan bei einem Verhältnis X'/T' unter 200 wobei diese Keime verschieden von dem Zeolith vom Strukturtyp EUO sind, den man herstellt.

2. Verfahren nach Anspruch 1, bei dem die Keime von zeolithischem Material einen von dem Strukturtyp EUO unterschiedlichen Strukturtyp haben.

3. Verfahren nach einem der Ansprüche 1 bis 2, derart, dass die Keime eine chemische Zusammensetzung des Kristallgitters haben, die unterschiedlich von der chemischen Zusammensetzung des Kristallgitters des Zeoliths vom Strukturtyp EUO ist, den man herstellt.

4. Verfahren nach einem der Ansprüche 1 bis 3, derart, dass das als Keime verwendete zeolithische Material, gewählt wird unter den Zeolithen vom Strukturtyp LTA, als Zeolith A vom Strukturtyp LTL, vom Strukturtyp FAU als die Zeolithen X und Y vom Strukturtyp MOR, vom Strukturtyp MAZ, vom Strukturtyp OFF, vom Strukturtyp FER, vom Strukturtyp ERI, vom Strukturtyp BEA, vom Strukturtyp MFI als ZSM-5 und das Silikat vom Strukturtyp MTW, vom Strukturtyp MTT, vom Strukturtyp LEV, vom Strukturtyp TON, vom Strukturtyp NES als der NU-87 Zeolith, die NU-85-, NU-86-, NU-88-, IM-5-Zeolithe oder die Zeolithe vom Strukturtyp EUO von einer chemischen Zusammensetzung des Kristallgitters, die unterschiedlich von dem EUO-Zeolithen ist, den man herstellt als die Zeolithe EU-1, ZSM-50 und TPZ-3.

5. Verfahren nach einem der Ansprüche 1 bis 4, derart, dass die verwendeten Keime gewählt werden unter Keimen von wenigstens einem zeolithischen Material vom Strukturtyp LTA, FAU, MOR, MFI und EUO.

6. Verfahren nach einem der Ansprüche 1 bis 5, in dem die Keime von zeolithischem Material an irgendeinem Moment der Herstellung eingeführt werden.

7. Verfahren nach einem der Ansprüche 1 bis 6, bei dem die Keime von zeolithischem Material nach Homogenisierung wenigstens eines Teils der wässrigen Mischung eingeführt werden, welche die Quelle vom Element X, die Quelle vom Element T, die Quelle organischen Strukturbildners enthält.

8. Verfahren nach einem der Ansprüche 1 bis 7, derart, dass das Element X Silizium und das Element T Aluminium ist.

9. Verfahren nach einem der Ansprüche 1 bis 8, derart, dass der organische Strukturbildner ein alkyliertes Derivat von Diammonium-$\alpha$-$\omega$-Polymethylen ist von einer Formel: $R_1R_2R_3 \ N^+ \ (CH_2)_n \ N^+ \ R_4R_5R_6$ und/oder ein Aminabbauprodukt, das dem Derivat entspricht und/oder ein Vorläufer, der dem Derivat entspricht, wobei n zwischen 3 und 12 liegt und $R_1$ bis $R_6$ identisch oder verschieden Alkyl- oder Hydroxyalkylradikale mit 1 bis 8 Kohlenstoffatomen darstellen können, wobei bis zu fünf der Radikalen $R_1$ bis $R_6$ Wasserstoff sein können.

10. Verfahren nach einem der Ansprüche 1 bis 8, derart, dass der organische Strukturbildner eine Verbindung ist, die Dibenzyldimethylammonium und/oder ein Aminabbauprodukt umfasst, das dem Derivat und/oder einem entsprechenden Vorläufer des Derivats entspricht.

11. Verfahren nach einem der Ansprüche 1 bis 10, derart, dass man wenigstens ein Alkalimetall- oder Ammoniumsalz P einführt.

12. Verfahren nach einem der Ansprüche 1 bis 9, bei dem das Reaktionsgemisch die folgende, in Oxydform ausgedrückte Zusammensetzung hat:

| | | |
|---|---|---|
| $XO_2/T_2O_3$ | (mol/mol) | wenigstens 10 |
| $OH^-/XO_2$ | (mol/mol) | 0,002 bis 2,0 |
| $Q/XO_2$ | (mol/mol) | 0,002 bis 2,0 |
| $Q/(M^+ + Q)$ | (mol/mol) | 0,1 bis 1,0 |
| $H_2O/XO_2$ | (mol/mol) | 1 bis 500 |
| $P/XO_2$ | (mol/mol) | 0 bis 5 |
| $S/XO_2$ | (g/g) | 0,0001 bis 0,1 |

wobei
X Silizium und/oder Germanium ist,
T wenigstens ein Element ist, gewählt unter Aluminium, Eisen, Gallium, Bor, Titan, Vanadium, Zirkonium, Molybdän, Arsen, Antimon, Chrom und Mangan.
$M^+$ ein Alkalimetall oder ein Ammoniumion darstellt,
Q den organischen Strukturbildner darstellt, oder die Zersetzungsprodukte, die dem Derivat entsprechen, oder den Vorläufern des Derivats.
S die in Rohform vorliegenden Zeolithkeime, getrocknet, kalziniert oder ausgetauscht mit einem Verhältnis X'/T' unter 200 darstellt.
P das Alkalimetall- oder Ammoniumsalz darstellt.

**Claims**

1. A process for preparing a zeolitic material with structure type EUO comprising mixing, in an aqueous medium, at least one source of at least one element X selected from silicon and germanium, at least one source of at least one element T selected from iron, aluminium, gallium, boron, titanium, vanadium, zirconium, molybdenum, arsenic, antimony, chromium and manganese, at least one source of an organic structuring agent, **characterized in that** seeds of at least one zeolitic material comprising at least one element X' selected from silica and germanium and at least one element T' selected from iron, aluminium, gallium, boron, titanium, vanadium, zirconium, molybdenum, arsenic, antimony, chromium and manganese is used with an X'/T' ratio of less than 200, said seeds being different from the zeolite with structure type EUO which is prepared.

2. A process according to claim 1, in which the structure type of the zeolitic material seeds is different from the EUO structure type.

3. A process according to claim 1 or claim 2, in which the chemical composition of the crystalline framework of the seeds is different from the chemical composition of the crystalline framework of the zeolite with structure type EUO which is prepared.

4. A process according to any one of claims 1 to 3, in which the zeolitic material used as seeds is selected from zeolites with structure type LTA such as A zeolite, structure type LTL, structure type FAU such as X and Y zeolites, structure type MOR, structure type MAZ, structure type OFF, structure type FER, structure type ERI, structure type BEA, structure type MFI such as ZSM-5 and silicalite, structure type MTW, structure type MTT, structure type LEV, structure type TON, structure type NES such as NU-87 zeolite, or a NU-85, NU-86, NU-88 or IM-5 zeolite or a zeolite with structure type EUO with a chemical composition of the crystalline framework which is different from the EUO zeolite which is prepared, such as EU-1, ZSM-50 and TPZ-3 zeolites.

5. A process according to any one of claims 1 to 4, in which the seeds used are selected from seeds of at least one zeolitic material with structure type LTA, FAU, MOR, MET or EUO.

6. A process according to any one of claims 1 to 5, in which the zeolitic material seeds are introduced at any point in the preparation.

7. A process according to any one of claims 1 to 6, in which the zeolitic material seeds are introduced after at least partial homogenisation of the aqueous mixture containing the source of element X, the source of element T and the source of the organic structuring agent.

8. A process according to any one of claims 1 to 7, in which the element X is silicon and the element T is aluminium.

9. A process according to any one of claims 1 to 8, in which the organic structuring agent is an alkylated polymethylene $\alpha$-$\omega$ diammonium derivative with formula: $R_1R_2R_3N^+(CH_2)_nN^+ R_4R_5R_6$ and/or an amine degradation product corresponding to said derivative and/or a precursor corresponding to said derivative, n being in the range 3 to 12 and $R_1$ to $R_6$, which may be identical or different, possibly representing alkyl or hydroxyalkyl radicals containing 1 to 8 carbon atoms, and up to five radicals $R_1$ to $R_6$ possibly being hydrogen.

10. A process according to any one of claims 1 to 8, in which the organic structuring agent is a compound comprising dibenzyldimethylammonium and/or an amine degradation product corresponding to said derivative and/or a precursor corresponding to said derivative.

11. A process according to any one of claims 1 to 10, in which at least one alkali metal or ammonium salt is introduced.

12. A process according to any one of claims 1 to 9, in which the reaction mixture has the following composition, expressed in the oxide form:

| | |
|---|---|
| $XO_2/T_2O_3$ (mol/mol) | at least 10 |
| $OH^-/XO_2$ (mol/mol) | 0.002 to 2.0 |
| $Q/XO_2$ (mol/mol) | 0.002 to 2.0 |
| $Q/(M^+ + Q)$ (mol/mol) | 0.1 to 1.0 |
| $H_2O/XO_2$ (mol/mol) | 1 to 500 |
| $P/XO_2$ (mol/mol) | 0 to 5 |
| $S/XO_2$ (g/g) | 0.0001 to 0.1 |

where
X is silicon and/or germanium,
T is at least one element selected from aluminium, iron, gallium, boron, titanium, vanadium, zirconium, molybdenum, arsenic, antimony, chromium and manganese;
$M^+$ represents an alkali metal or an ammonium ion;
Q represents the organic structuring agent or decomposition products corresponding to said derivative or precursors of said derivative;
S represents zeolite seeds expressed in their dried as synthesised, calcined or exchanged form with an X'/T' ratio of less than 200;
P represents an alkali metal or ammonium salt.